Europäisches Patentamt

(19) European Patent Office

Office européen des brevets

(11) Numéro de publication: **0 069 104**
**B1**

(12) **FASCICULE DE BREVET EUROPEEN**

(45) Date de publication du fascicule du brevet:
**17.04.85**

(21) Numéro de dépôt: **82870031.0**

(22) Date de dépôt: **10.06.82**

(51) Int. Cl.⁴: **A 61 M 15/02, A 61 B 5/08**

(54) **Appareil pour réaliser un examen de ventilation pulmonaire au moyen d'aérosols radioactifs.**

(30) Priorité: **24.06.81 LU 83455**

(43) Date de publication de la demande:
**05.01.83 Bulletin 83/1**

(45) Mention de la délivrance du brevet:
**17.04.85 Bulletin 85/16**

(84) Etats contractants désignés:
**AT BE CH DE FR GB IT LI NL SE**

(56) Documents cités:
**DE - C - 270 862**

**ATOMKERNENERGIE (ATKE), vol.26, no.2, 1975, Verlag K. Thiemig A.G., Munich (DE) W.PUSCH:"Ein Versuchsaufbau für Inhalationsexperimente mit radioaktiven Aerosolen",pages 125-128**

(73) Titulaire: **INSTITUT MEDICAL SPECIALISE S.A., Route de Mons 230, B-7320 Hornu (BE)**

(72) Inventeur: **Godart, G., rue d'Hautrage 23, B-7420 Baudour (BE)**

(74) Mandataire: **Van Malderen, Michel et al, p.a. Freylinger & Associés 22 avenue J.S. Bach (bte 43), B-1080 Bruxelles (BE)**

## Description

La présente invention concerne un appareil pour réaliser un examen de ventilation pulmonaire au moyen d'aérosols radioactifs.

L'étude de la perfusion pulmonaire est aujourd'hui devenue classique et routinière dans tous les laboratoires de diagnostic radioisotopique.

Par contre, l'étude de la ventilation pulmonaire par scintigraphie n'a pas connu le même essor et de nombreux laboratoires renoncent à utiliser cette technique malgré l'intérêt évident que présente la confrontation des résultats obtenus par la scintigraphie de perfusion et de ventilation, notamment pour le diagnostic de troubles embolitiques.

Ce manque d'enthousiasme des laboratoires à propos de l'utilisation de la technique de l'examen de la ventilation pulmonaire par inhalation d'air chargé d'un radioisotope suivie d'un prise de vue par une gamma-caméra provient de certains inconvénients dus aux propriétés des gaz radioactifs habituellement utilisés, en général le xénon 133 ou le krypton 81 et aux prescriptions réglementant leur utilisation.

Le xénon 133, qui peut être employé par inhalation continue ou par inhalation d'une bouffée unique, est très aisément disponible à tout moment au lieu de l'utilisation, mais il présente l'inconvénient, en raison de son très faible taux de décroissance, de donner des images difficiles à interpréter du fait que l'activité mesurée peut être imputable non seulement au débit de la ventilation mais aussi au volume d'air intrathoracique. Pour éviter cet inconvénient, on peut effectuer un rinçage complet à l'air ambiant (wash-out) après chaque incidence, mais le temps nécessaire à l'examen et, par conséquent, son coût s'en trouvent multipliés.

Le krypton 81 a un taux de décroissance beaucoup plus élevé que celui du xénon et ne présente donc pas l'inconvénient ci-dessus, c'est-à-dire qu'il permet toujours de mesurer l'importance de la ventilation indépendamment du volume d'air intratoracique. Malheureusement il constitue un produit de la désintégration du rubidium 81, qui est lui-même un produit de cyclotron, et sa demi-vie physique n'est que de 5 heures environ; il en résulte qu'il n'est pas toujours possible de s'en procurer sur le lieu et au moment où on doit l'utiliser. En outre, ce gaz est relativement onéreux.

Pour remédier à ces inconvénients, on a déjà songé à utiliser des aérosols radioactifs au lieu de gaz radioactifs. Toutefois, les résultats de la scintigraphie sont beaucoup moins satisfaisants du fait qu'en général les nuages créés par le nébuliseur sont polydispersés et que les micelles sont moins aptes à pénétrer profondément dans les ramifications broncho-pulmonaires. On constate, en effet, que les particules radioactives inhalées ont tendance à s'accumuler en amont des obstacles tels que les sténoses, les corps étrangers ou les amas de secrétion, en formant des accumulations indésirables.

Il convient donc, pour permettre d'utiliser efficacement les aérosols marqués, de chercher un moyen d'éviter ces accumulations et de faciliter la pénétration des particules dans l'arbre bronchique.

Comme les particules aérosolisées sont en mouvement, elles possèdent un moment d'inertie MI qui est égal au produit de la masse par la vitesse:

$$MI = m \cdot v$$

On sait aussi que le tendance à l'accumulation susdite est d'autant plus grande que le moment d'inertie est plus élevé. On a donc intérêt à agir sur les éléments constitutifs de ce moment, c'est-à-dire la masse ou la vitesse des particules ou des micelles, pour réduire l'importance de celui-ci.

La présente invention part du principe qu'il est plus aisé d'abaisser le moment d'inertie des particules en agissant sur leur masse plutôt que sur la vitesse et le moyen utilisé à cet effet consiste à soumettre les aérosols, avant leur inhalation, à un processus de sédimentation. De cette manière, les grosses particules sédimentent tandis que celles plus faibles dimensions, par conséquent de masse moindre, sont plus facilement entraînées par le flux d'air et pénètrent plus profondément dans l'arbre bronchique du patient.

La présente invention concerne un appareil pour réaliser un examen de ventilation pulmonaire au moyen d'aérosols radioactifs caractérisé en ce qu'il comporte un boîtier blindé contenant:

— un nébuliseur de liquide radioactif relié d'une part à une source extérieure d'air sous pression et d'autre part à une conduite d'amenée de l'aérosol formé dans le nébuliseur vers une valve »inspiration-expiration«, munie d'un embout buccal,
— des zones de sédimentation de l'aérosol branchées sur ladite conduite d'amenée entre le nébuliseur et l'embout,
— une conduite partant de l'embout pour le transport vers l'extérieur de l'air expiré,
— un dispositif de réception de l'air expiré placé en aval de l'embout et en amont d'une pompe destinée à la purge du dispositif de réception de l'air expiré et à l'évacuation des résidus des zones de sédimentation de l'aérosol après la fin du test,
— une série de valves à faible réduction de calibre, placées en amont, en aval et sur le branchement de chacune des zones de sédimentation du circuit inspiratoire et en amont du dispositif de réception de l'air expiré, permettant un accès sélectif séquencé à chacune des parties de l'appareil, notamment à chacune des zones de sédimentation de l'aérosol et au dispositif de réception de l'air expiré, au cours des diverses phases du fonctionnement.

Suivant une forme préférée de réalisation de l'invention, les zones de sédimentation de l'aérosol consistent en des sacs suspendus dans le boîtier.

Le dispositif de réception peut être un sac ou un filtre adéquat, notamment un filtre de type VOKES®, MK 2.

Il va de soi qu'il est avantageux de faciliter l'utilisation de l'appareil en employant des valves automatiques éventuellement commandées par un microprocesseur plutôt que des valves à ouverture manuelle.

De plus, comme l'appareil est utilisé de la même manière pour tous les patients, on peut avantageusement l'associer à un microprocesseur réalisant de manière automatique l'ouverture et la fermeture des valves dans la séquence nécessaire pour l'administration de l'aérosol au patient et pour la vidange des zones de sédimentation et la purge du dispositif de réception.

Enfin, suivant une forme particulière d'exécution, le boîtier contient encore — en aval de la pompe — un filtre à petites particules dans lequel passe l'air à évacuer.

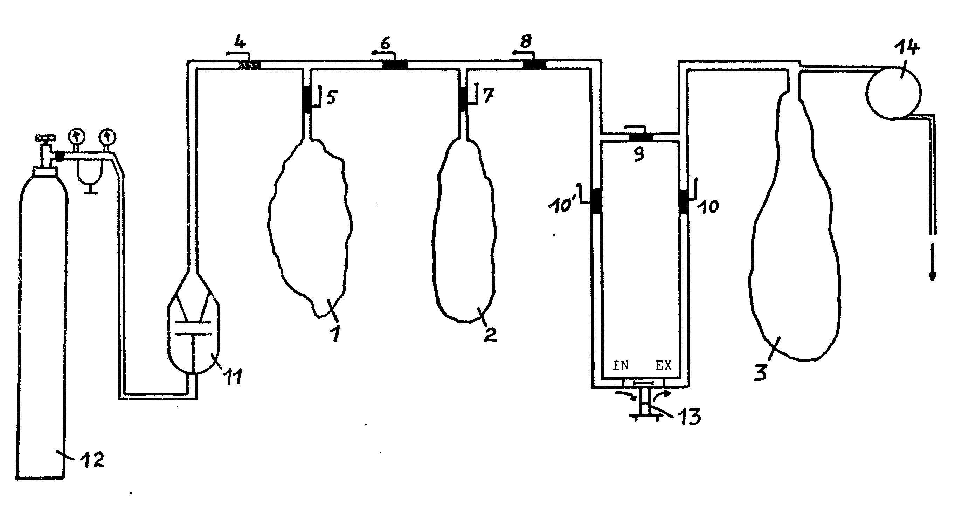

Pour mieux expliciter l'invention, on va en donner à présent un exemple de réalisation non limitatif en se référant à la figure unique annexée.

L'appareil représenté schématiquement à la figure se compose d'un boîtier blindé par une feuille de plomb (non illustré) contenant deux sacs de DOUGLAS de 50 litres, 1 et 2, que constituent les zones de sédimentation, et un sac de 100 litres, 3, pour le recueil de l'air expiré. Ces sacs sont suspendus et reliés entre eux par une conduite pourvue de toute une série de valves 4, 5, 6, 7, 8, 9, 10 permettant un accès sélectif à chacun d'eux.

Ces valves sont d'un type tel qu'en position ouverte la réduction du calibre soit minimale afin de ne pas introduire de résistance exagérément élevée dans les circuits.

Un emplacement spécial, avec renforcement du blindage, est aménagé pour recevoir le nébuliseur proprement dit 11. Celui-ci est relié au détendeur d'une bouteille d'air sous pression 12.

Les deux sacs de 50 litres 1, 2 servent à recueillir l'aérosol et à la sédimentation de celui-ci. Ils font partie du circuit inspiratoire.

Le patient est relié à ce circuit par l'intermédiaire d'un embout, 13, inspiratoire-expiratoire, type valve de RUBEN.

Le sac de 100 litres, 3, fait partie du circuit expiratoire et permet de recueillir l'air expiré par le patient. Ce sac 3 évite donc toute contamination des locaux.

Le fait d'utiliser un ballon 3 de recueil de l'air expiré et un blindage complet de la caisse (qui est montée sur roulettes) permet de réaliser l'examen, à n'importe quel endroit du laboratoire et même devant la gamma-caméra sans risque de perturber les images par un éventuel bruit de fond trop élevé.

Une valve de sécurité 10 est montée dans le circuit expiratoire. Cette valve a pour fonction d'empêcher un retour éventuel de l'air expiré recueilli par le ballon 3 vers la valve respiratoire 13. Cette valve 10 sert donc à éviter la contamination des locaux lorsque le patient est déconnecté du circuit.

Le circuit expiratoire se termine par une pompe 14 qui permet la vidange du sac expiratoire 3, soit vers une hotte munie de filtres adéquats, soit directement vers un filtre à petites particules, type VOKES®, (non représenté) qui peut éventuellement être monté dans le boîtier.

Un court-circuit muni d'une valve 9 est réalisé entre le circuit inspiratoire et le circuit expiratoire. Ce court-circuit a pour fonction de vidanger les deux sacs inspiratoires 1, 2 qui ne sont jamais vidés complètement par les patients testés.

La technique d'utilisation de cet appareil est la suivante.

Après avoir introduit dans le nébuliseur 11 le produit que l'on désire aérosoliser au patient (par exemple de l'acide diéthylène-triamine pentacétique marqué au technétium 99), on ouvre les valves 4 et 5, la valve 6 étant fermée; on réalise ainsi le remplissage du sac 1. Le débit de l'air est, par exemple, réglé sur 12 à 15 litres par minute afin que le remplissage s'effectue en un laps de temps de l'ordre de 4 à 5 minutes.

Après avoir renouvelé la solution du nébuliseur, on ouvre les valves 4, 6 et 7 et on ferme les valves 5 et 8 pour remplir le sac 2. Pendant ce temps, l'aérosol créé dans le sac 1 sédimente.

Lorsque le sac 2 est rempli, on ferme les valves 6, 4.

On relie alors le patient à l'appareil et on ouvre les valves 5, 6, 8, 10, les autres valves étant fermées.

Pendant que le patient respire le contenu du sac 1, l'aérosol du sac 2 a le temps de sédimenter.

Lorsque le patient a respiré environ les 3/4 du contenu du sac 1, on ferme les valves 5, 6 et on ouvre la valve 7. Le patient respire alors le contenu du sac 2 sans qu'il soit nécessaire de le déconnecter du circuit.

Pendant tout ce temps, le patient expire dans le sac 3 qui se remplit lentement.

Lorsque le patient a vidé les 3/4 du contenu du sac 2, on ferme les valves 8 et 10. A ce moment, la personne testée peut passer à la gamma-caméra et on peut commencer les acquisitions des images de ventilation.

Dès que l'aérolisation est terminée, ou même un peu avant, on met la pompe 14 en fonctionnement. Cette pompe a pour fonction de vider le sac 3. Lorsque ce sac est vide, on ouvre les valves 5, 6, 7, 8 et 9 afin d'éliminer les résidus des sacs 1 et 2.

Il est évident que ce mode opératoire peut être facilité par l'emploi de valves électriques.

En outre, comme il s'agit d'un cycle qui se renouvelle de la même façon pour chaque patient, on peut avantageusement envisager lors de la construction de l'appareil l'emploi d'un microprocesseur programmé pour réaliser automatiquement les différentes séquences nécessaires à l'administration de l'aérosol au patient. Il suffirait alors d'appuyer sur un seul bouton pour mettre tout le cycle en œuvre.

Bien que l'invention ait été décrite par un exemple détaillé, il est évident qu'elle n'est pas limitée à cette forme de réalisation, et qu'elle comprend également, dans sa portée, toutes les modifications ou variantes accessibles à un expert de la branche pourvu qu'elles relèvent de l'idée inventive.

On peut notamment substituer à la bonbonne 12, un compresseur d'air et intercaler notamment dans la branche entre la valve 8 et la valve respiratoire 13, parallèle à la branche comportant la valve 10, une valve 10' pour éviter des perturbations au niveau de la valve respiratoire 13. Il est également possible de remplacer le sac 3 par un filtre en amont de la pompe 14, ce qui n'exclut pas cependant de monter un second filtre de sécurité, en aval de cette pompe.

## Revendications

1. Appareil pour réaliser un examen de ventilation pulmonaire au moyen d'aérosols radioactifs caractérisé en ce qu'il comporte un boîtier blindé contenant:

— un nébuliseur (11) de liquide radioactif relié d'une part à une source extérieure d'air sous pression (12) et d'autre part à une conduite d'amenée de l'aérosol formé dans le nébuliseur (11) vers un embout d'inspiration-expiration (13),

— des zones de sédimentation (1, 2) de l'aérosol branchées sur ladite conduite d'amenée entre le nébuliseur (11) et l'embout (13),

— une conduite partant de l'embout (13) pour le transport vers l'extérieur de l'air expiré,

— un dispositif de réception (3) de l'air expiré placé en aval de l'embout (13) et en amont d'une pompe (14) destinée à la purge du dispositif de réception (3) de l'air expiré et à l'évacuation des résidus des zones de sédimentation (1, 2) de l'aérosol après la fin du test,

— une série de valves (4, 5, 6, 7, 8, 9, 10, 10') à faible réduction de calibre, placées en amont, en aval et sur le branchement de chacune des zones de sédimentation (1, 2) du circuit inspiratoire et en amont du dispositif de réception (3) de l'air expiré, permettant un accès sélectif séquencé à chacune des parties de l'appareil, notamment à chacune des zones de sédimentation (1, 2) de l'aérosol et au dispositif de réception (3) de l'air expiré, au cours des diverses phases du fonctionnement.

2. Appareil suivant la revendication 1 caractérisé en ce que les zones de sédimentation de l'aérosol consistent en des sacs (1, 2) suspendus dans le boîtier.

3. Appareil suivant la revendication 1 caractérisé en ce que le dispositif de réception de l'air expiré consiste en un sac (3) suspendu dans le boîtier.

4. Appareil suivant la revendication 1 caractérisé en ce que le dispositif de réception de l'air expiré consiste en un filtre.

5. Appareil suivant l'une quelconque des revendications 1 à 4 caractérisé en ce que les valves sont commandées électriquement.

6. Appareil suivant l'une quelconque des revendications 1 à 4 caractérisé en ce qu'il est associé à un microprocesseur qui réalise de manière automatique l'ouverture et la fermeture des valves dans la séquence nécessaire pour l'administration de l'aérosol au patient et pour la vidange des zones de sédimentation et du dispositif de réception de l'air expiré.

7. Appareil suivant l'une quelconque des revendications précédentes caractérisé en ce que le boîtier contient en outre, en aval de la pompe, un filtre à petites particules dans lequel passe l'air à évacuer.

## Patentansprüche

1. Gerät zum Untersuchen der Lungenventilation mittels radioaktiver Aerosole, dadurch gekennzeichnet, daß es ein abgeschirmtes Gehäuse enthält, in dem

— ein Verstäuber (11) von radioaktiver Flüssigkeit untergebracht ist, der einerseits an eine außen angebrachte Druckluftquelle (12) und andererseits an eine Zufuhrleitung des im Verstäuber (11) gebildeten Aerosols zu einem Mundstück zum Ein- und Ausatmen (13) verbunden ist,

— Zonen zur Aerosol-Niederschlagsbildung (1, 2) vorgesehen sind, die an die genannte Zufuhrleitung, zwischen Verstäuber (11) und Mundstück (13) angeschlossen sind,

— eine vom Mundstück (13) ausgehende Leitung zum Befördern der Luft nach außen hin vorgesehen ist,

— eine Vorrichtung (3) zur Aufnahme der ausgeatmeten Luft untergebracht ist, die hinter dem Mundstück (13) und vor einer Pumpe (14) geschaltet ist, die zum Entlüften der Vorrichtung zur Aufnahme der ausgeatmeten Luft und zum Entfernen der Niederschläge aus den Zonen der Aerosol-Niederschlagsbildung (1, 2) nach dem Test dient,

— eine Anordnung von Ventilen (4, 5, 6, 7, 8, 9, 10, 10') mit geringer Bohrungsverminderung vorgesehen ist, die vor, hinter und in der Abzweigung einer jeden Zone zur Niederschlagsbildung (1, 2) des Einatmungskreislaufes, und vor der Vorrichtung (3) zur Aufnahme der ausgeatmeten Luft geschaltet sind, wobei diese Ventile einen selektiven sequentiellen Zugang zu jeden Geräteteil, insbesondere zu jeder Zone zur Aerosol-Niederschlagsbildung (1, 2) und zu der Vorrichtung (3) zur Aufnahme der ausgeatmeten Luft, während der verschiedenen Betriebsstadien ermöglichen.

2. Gerät nach Anspruch 1, dadurch gekennzeichnet, daß die Zonen zur Aerosol-Niederschlagsbildung aus in dem Gehäuse aufgehangenen Beuteln (1, 2) besteht.

3. Gerät nach Anspruch 1, dadurch gekennzeichnet, daß die Vorrichtung zur Aufnahme der ausgeatmeten Luft aus einem in dem Gehäuse aufgehangenen Beutel (3) besteht.

4. Gerät nach Anspruch 1, dadurch gekennzeichnet, daß die Vorrichtung zur Aufnahme der ausgeatmeten Luft aus einem Filter besteht.

5. Gerät nach einem der Ansprüche 1 bis 4, dadurch gekennzeichnet, daß die Ventile elektrisch betätigt werden.

6. Gerät nach einem der Ansprüche 1 bis 4, dadurch gekennzeichnet, daß es mit einem Mikroprozessor verbunden ist, der automatisch das Öffnen und Schließen der Ventile in der bestimmten Folge betätigt, die es erlaubt, dem Patienten das Aerosol zu verabreichen, und die Zonen zur Niederschlagsbildung sowie die Vorrichtung zur Aufnahme der ausgeatmeten Luft zu entleeren.

7. Gerät nach einem der vorhergehenden Ansprüche, dadurch gekennzeichnet, daß das Gehäuse außerdem, hinter der Pumpe, einen Filter für kleine Teilchen enthält, der durch die auszutragende Luft durchströmt wird.

**Claims**

1. Lung ventilation examination apparatus using radioactive aerosols characterized in that it comprises a protected housing including

— a spray (11) of radioactive liquids, which is connected, on the one hand, to an external pressurized air source (12) and on the other hand, to a delivery pipe which supplies the aerosol formed in the spray (11) to an adapter (13) for inspiring and expiring;
— aerosol sedimentation zones (1, 2), said zones being connected to said delivery pipe, between the spray (11) and the adapter (13);
— a conduct starting from the adapter (13), intended for transfering the expired air to the outside;
— an expired air reception means (3) disposed on the downstream side of the adapter (13) and on the upstream side of a pump (14) intended for blowing-off of the expired air reception means (3) and for the discharge of the residues contained in the zones for sedimentation (1, 2) of the aerosol, after the end of the test;
— a series of valves (4, 5, 6, 7, 8, 9, 10, 10') having a small size reduction and being placed on upstreamside, on downstream-side and on the branch-line of each sedimentation zone of the inspiratory circuit and on upstream side of the expired air reception means (3), allowing a selective sequential accesc to each part of the apparatus, particularly to each aerosol sedimentation zone (1, 2) and to the expired air reception means (3), during the different operating stages.

2. Apparatus according to claim 1 characterized in that the aerosol sedimentation zones consist in sacks (1, 2) suspended in the housing.

3. Apparatus according to claim 1 characterized in that the expired air reception means consists in a sack (3) suspended in the housing.

4. Apparatus according to claim 1 characterized in that the expired air reception means consists in a filter.

5. Apparatus according to anyone of claims 1—4 characterized in that the valves are electrically controlled.

6. Apparatus according to anyone of claims 1—4 characterized in that it is associated to a micro-processor which automatically controls the opening and the closing of the valves in the sequence which is necessary for the administration of the aerosol to the patient and for the blowing-off of the sedimentation zones and of the expired air reception means.

7. Apparatus according to anyone of the preceding claims, characterized in that the housing further includes, on the downstream side of the pump, a filtre for small particles wherethrough the air to be eliminated is passed.

0 069 104